(19) Europäisches Patentamt
European Patent Office
Office européen des brevets

(11) Publication number: 0 002 872 A1

# (12) EUROPEAN PATENT APPLICATION

(21) Application number: 78200388.3

(22) Date of filing: 21.12.78

(51) Int. Cl.2: C 07 C 69/90, C 07 C 67/10, C 07 C 67/14, C 07 C 67/08 // C07C69/88

(30) Priority: 23.12.77 US 864033

(43) Date of publication of application: 11.07.79 Bulletin 79/14

(84) Designated Contracting States: BE CH DE FR GB IT LU NL SE

(71) Applicant: THE PROCTER & GAMBLE COMPANY, 301 East Sixth Street, Cincinnati Ohio 45217 (US)

(72) Inventor: Buckwalter, Brian Lee, 621 Glensprings Dr., Cincinnati OH 45246 (US)
Inventor: Kretschmar, Herbert Charles, 160 Julep Lane, Cincinnati OH 45218 (US)

(74) Representative: Ernst, Hubert (BE,NL) et al, Procter & Gamble European Technical Center 100, Temselaan, B-1820 Strombeek-Bever (BE)

## (54) Process for preparing acylated esters of hydroxy carboxylates.

(57) A process for esterifying and acylating hydroxycarboxylate compounds in an organonitrile reaction medicine comprising the steps of contacting said compounds with an esterification agent and thereafter acylating by adding an acylating agent to the reaction medium.

EP 0 002 872 A1

# PROCESS FOR PREPARING ACYLATED ESTERS OF HYDROXY CARBOXYLATES

The present invention relates to methods for preparing acyl esters of hydroxy-substituted carboxylic acids. Acylated esters, especially acetylated gentisic acid esters, are particularly useful anti-inflammatory and analgesic agents. Because of their usefulness, there is a continuing search for more efficient methods for preparing these types of compounds.

Efficient reactions to esterify carboxylic acids and efficient reactions to acylate hydroxycarboxylic acids have been achieved before. However, the preparation of mixed esterified/acylated carboxylic acid derivatives has continued to be a laborious process because intermediate isolation or purification steps are required before the final product is secured. For example, an esterification reaction can take place in aqueous media, but for a subsequent acylation reaction to be efficient in producing an acylated product without byproducts or impurities, the acylation must be carried out in nonaqueous media. For that reason, the intermediate ester compound needs to be purified and dried before the acylation reaction is initiated.

Similarly, if the acylation reaction occurs first, the product must be purified to remove acidic reaction byproducts, such as the free acids produced by the reaction of a hydroxyl group with an acid halide or acid anhydride, before a subsequent esterification reaction can be performed efficiently. Moreover, the esterification reaction often requires that an intermediate acylated acid halide be prepared and purified.

In brief, the preparation of acylated esters of carboxylic acids using standard synthetic techniques is inconvenient and laborious because of the need to prepare and isolate reaction intermediates and to change solvent systems mid-way in the process.

The present process is a two-step, one-pot synthesis of acyl-carboxylate-esters, especially acyl-esters of aryl hydroxycarboxylic acids. The two steps of the present reaction sequence occur consecutively in the same reaction mixture; no purification of intermediate compounds between the two steps is necessary and no special, rigorous handling precautions need be taken. These desirable results are achieved by using organonitriles as the reaction medium.

Related References

It is recognized that solvents can be divided into three general classes. Solvents having dielectric constants of 15 or higher but without strong hydrogen bonding capabilities, e.g. acetonitrile, are classified as dipolar aprotic solvents, in contrast with protic solvents which have high dielectric constants and hydrogen bonding characteristics, e.g. water, and inert solvents which have low dielectric constants and hydrogen bonding tendencies, e.g. xylene. It is also well known that substitution reactions are faster and cleaner in dipolar aprotic solvents than they are in protic solvents. Parker, Chem. Tech. 1, 297, (1971); Parker, Chem. Rev. 69, 1 (1969); Miller, et al., JACS 83, 117 (1961).

It is also recognized that esterification reactions using some types of organohalides, such as benzyl chloride, may take place in aqueous solution. The aqueous reaction of benzyl chloride with carboxylic acid salts such as sodium benzoate to produce esters, such as benzylbenzoate, is disclosed by Gomberg, et al., JACS, 42, 2059 (1920) and Rueggeberg, et al., Ind. and Eng. Chem. 38, 207 (1946).

The use of dipolar aprotic solvents as the reaction medium for esterification reactions is also well known, see U.S. Patent 3,048,600, Jaruzelski, August 7, 1962 (dimethylformamide); U.S. Patent 3,069,459, Tsou, et al., December 18, 1962 (acetamide); Huang, et al., Ind. & Eng. Chem. 8, 227 (1969) (nitrobenzene); Pfeffer, et al., Tetra. Ltrs. 4063 (1972) ethanol/hexamethylphosphoramide); Dockx, Synthesis, 441 (1973) (acetone); Pfeffer, et al., J. Org. Chem. 41, 1373 (1976) (N-methylpyrrolidone, dimethylsulfoxide, hexamethylphosphoramide, dimethylformamide).

The use of amines and hydroxides to increase the rate of reaction in esterifications using alkyl chlorides and carboxylic acids is also well known. See German Patent 268,621, B.A.S.F., December 22, 1913; Volwiler, et al., JACS 43, 1672 (1921); Rueggeberg, et al., cited above; Merker, et al., J. Org. Chem. 26, 5180 (1961); Shaw, et al., Tetra. Ltrs. 689, (1973); Dockx (cited above).

Attention is directed to two references which relate to esterification procedures similar to the first step of the instant process. Wagenknecht, et al., Synth. Com. 2, 215 (1972) disclose a "Rapid Mild Esterification Method". The process involves the room temperature reaction of a preformed quaternary ammonium salt of a carboxylic acid with an alkyl halide to produce the ester. The article reveals that the use of a quaternary ammonium salt, instead of a tertiary ammonium salt, increases the rate of the reaction. The reference also discloses that the identity of the solvent used "does not seem to be too critical" as long as it is "non-hydroxylic"; dimethylsulfoxide, dimethylformamide, and acetonitrile are specifically mentioned.

The second article is by Ho, et al., J.C.S. Chem. Com. 224, (1973). Ho discloses a method for the formation of methylthiomethyl esters involving the reaction of a carboxylic acid, triethylamine, and methylthiomethylchloride in refluxing acetonitrile.

Workers in the art do not appear to have appreciated the use of inorganic solvents, especially the organonitriles, in esterification reactions, especially for commercial production. Recently issued U.S. Patent 4,052,438, Papenfuhs, October 4, 1977, for example, discloses a process for preparing aromatic

hydroxycarboxylic acid alkyl esters. The patentee discloses that the process was developed for aqueous solution because of the difficulties in using solvents such as acetone (which is dipolar aprotic) on an industrial scale. The products of the Papenfuhs process would, of course, have to be purified before undergoing an acylation reaction such as Step 2 of the present process.

Processes similar to the second step of the instant process are also well known for making acylated esters of phenols. Harris, J. Pharm. Ass., 24, 553 (1935), discloses the reaction of acid chlorides and phenols in toluene, benzene or pyridine to produce acylated compounds. German Offen. 2,320,945, Giudicelli, November 8, 1973, describes the acetylation reaction of hydroxysalicylic acids with acetic anhydride in pyridine. Belgian Patent 520,872, Resex, July 15, 1953, describes a two-step acetylation process: acetic acid and thionyl chloride are reacted to yield acetyl chloride and acetic anhydride which are then further reacted with a phenol to give the acetylated phenol. More recently, U.S. Patent 3,821,401, Lacefield, June 28, 1974, describes the reaction of phenol, acid chloride, and acid anhydride in the presence of pyridine to give the acylated phenol.

French Patent 2,245,346, Synthelabo, May 30, 1975, and British Patent 1,393,326, Synthelabo, May 7, 1975, disclose a method for preparing acetoxybenzoic acid esters. A reading of the disclosures, however, shows that the patentees did not recognize the present solution to the problem of eliminating separation and purification steps. The processes disclosed involve esterification in dimethylformamide, removal of the solvent by reduced pressure evaporation, and acetylation of the residue with acetic anhydride or chloroacetone and pyridine. Thus, an intermediate separation step is required.

From the foregoing it can be appreciated that processes similar to the esterification, Step 1, and processes similar to the acylation, Step 2, of the present process are independently known to be useful for producing esters and acylated compounds, respectively. However, no workers in the art appear to have appreciated that the esterification reaction of Step 1 herein proceeds in such a clean, efficient manner in an organonitrile medium that the acylation reaction of Step 2 can be carried out in the same reaction medium to produce a final product of high purity without intervening separation or purification steps to remove either intermediate products or solvent.

## Summary of the Invention

The process herein is illustrated by the esterification of gentisic acid with benzyl bromide to form (I), the benzyl gentisate, followed by the acylation of (I) with acetyl bromide to form (II), benzyl 2,5-diacetoxybenzoate.

STEP 1

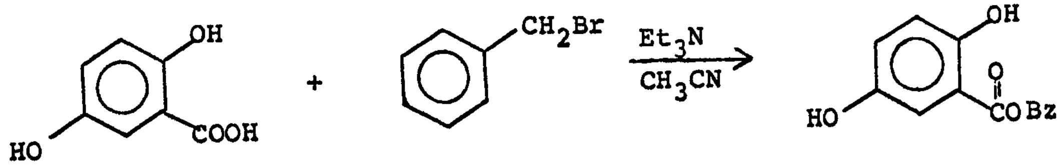


Gentisic Acid

(I)

STEP 2

Final reaction mixture of Step 1, including (I) and $CH_3CN$

+

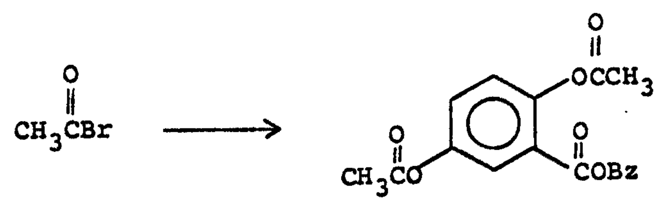

(II)

As illustrated by the foregoing reaction sequence, the present invention encompasses a process for preparing acylated esters of hydroxycarboxylate compounds, including hydroxycarboxylic acids and salts of hydroxycarboxylic acids, especially aryl hydroxycarboxylic acids. The process for preparing acylated esters of hydroxycarboxylate compounds comprises: Step 1, contacting a hydroxycarboxylic acid salt or a mixture of a hydroxycarboxylic acid and base with an esterification agent in a substantially water-free organonitrile reaction medium; and, Step 2, acylating the hydroxycarboxylate ester produced by Step 1 by admixing the acylating agent with the final reaction mixture of Step 1. If the acylating agent is an acid anhydride, an appropriate base is also added in this second step; if it is an acid halide, addition of an appropriate base in Step 2 is optional, although desirable.

The reaction herein can be carried out using hydroxycarboxylic acids or their alkali metal, amine or ammonium salts. If a hydroxycarboxylic acid in the free acid form is to be used, an alkali metal hydroxide, ammonium hydroxide, or amine base is included in the reaction mixture of Step 1. If a salt of a hydroxycarboxylic acid is used, it is preferably a tertiary or quaternary ammonium salt or an alkali metal salt, in which case, no additional base need be used in Step 1.

The process is simply and economically carried out by admixing the hydroxycarboxylic acid-plus-base, or hydroxycarboxylic acid salt, with the esterification agent in an organonitrile reaction medium; heating to reflux is usually employed to allow the esterification reaction to proceed to completion. Since there are only trace amounts

of water present in the reaction mixture and since there are few contaminating byproducts, the resulting reaction mixture containing the ester is then conveniently subjected to acylation in the same reaction medium to produce a high purity product.

DETAILED DESCRIPTION OF THE INVENTION

The present invention is a process for preparing acylated esters of hydroxycarboxylic acids and hydroxycarboxylic acid salts which comprises the steps of: (1) contacting the hydroxycarboxylate compound with an esterification agent in a substantially water-free organonitrile reaction medium, and, thereafter, (2) acylating the product of Step (1) by adding an acylating agent to the substantially water-free reaction medium of Step (1). Following Step (2), the esterified/acylated product can be secured by any convenient means. The hydroxycarboxylate compounds employed in the present reaction can be a base plus any organic acid having one or more hydroxyl and one or more carboxyl groups, or a preformed salt of a base and an organic acid having one or more hydroxyl and one or more carboxyl groups. The reaction does not appear to depend on the nature of the acid used as long as it is a compound having at least one carboxyl group and one hydroxyl functional group which can be acylated by acid anhydrides or acid halides.

By "hydroxycarboxylic acid" as used herein is meant a compound of the formula RCOOH wherein R can be any organic

substituent having at least one hydroxyl group, and which can also contain other carboxyl groups. By "aryl carboxylic acid" is meant an unsaturated carboxylic acid with an aromatic ring structure directly connected to a carboxyl group and by "hydroxy-substituted aryl carboxylic acid" and "aryl hydroxycarboxylic acid" are meant aromatic carboxylic acids with one or more hydroxyl groups attached at some point. Hydroxy-substituted aryl carboxylic acids have at least one hydroxyl group attached to the aromatic ring. Preferred aryl hydroxycarboxylic acids are hydroxybenzoic acids having from 1 to 5 hydroxyl groups and the most preferred acids are 2-hydroxybenzoic acid and 2,5-dihydroxybenzoic acid.

By "base" as used herein is meant compounds such as amine bases, alkali metal hydroxides, and quaternary ammonium hydroxides of the formula $NR^1R^2R^3R^4OH$, where $R^1$, $R^2$, $R^3$, and $R^4$ are organic substituents. It is known that compounds such as tertiary arsines and tertiary phosphines may also be used to promote the esterification reaction of Step 1. These types of compounds, however, are not contemplated for use in the instant process as they are very toxic and the products of the process disclosed herein are most desirably compounds which can be used in living organisms. The bases preferred for use are $C_1 - C_5$ alkyl amines, quaternary ammonium hydroxides, and alkali metal hydroxides and are most preferably $C_1 - C_5$ trialkylamines, especially trimethylamine, triethylamine, and tripropylamine, sodium and potassium hydroxide, and $C_1 - C_5$ alkyl quaternary ammonium hydroxides wherein $R^1$, $R^2$, $R^3$ and $R^4$ are alkyl groups which contain from 1 to 5 carbon atoms, especially tetramethyl-, tetraethyl-, tetrapropyl-, and tetrabutylammonium hydroxide.

By "amine bases" as used herein is meant primary, secondary, or tertiary amines, compounds of the formula $R^5R^6R^7N$ where $R^5$, $R^6$, and $R^7$ can be hydrogen or organic substituents. Alkylamines where one or more of the substituents is an aliphatic group can be used. Examples include octylamine, dipentylamine, trimethylamine, and tributylamine. Cycloalkylamines where one or more of the organic substituents is an alicyclic group such as cyclopropyl, cyclopentyl, or cyclooctyl can be used to promote the esterification reaction. Monoaryl amines wherein the nitrogen is directly attached to an aromatic ring structure, which can have organic substituents, can also be used. Examples are N,N-methylphenylamine, aniline, and 4-methylaniline. Heterocyclic and substituted heterocyclic amines in which the amine nitrogen is incorporated into a ring structure such as in pyridine, pyrrolidine, and piperdine are also used.

Amine bases can be used to promote the esterification reaction of Step 1, or used as bases to form hydroxycarboxylic acid salts which can then be used in Step 1. Preferred amine bases are $C_1$ - $C_5$ alkylamines wherein the alkylamine is a mono, di, or tri-substituted amine and the alkyl groups contain from 1 to 5 carbon atoms. Preferred $C_1$ to $C_5$ alkyl amine bases are tri-substituted amines wherein all three substituents are alkyl groups, each containing from 1 to 5 carbon atoms, especially triethylamine.

By "hydroxycarboxylic acid salt" as used herein is meant a compound resulting from the reaction of a base and a hydroxycarboxylic acid as those compounds are described above. The preferred salts of hydroxycarboxylic acids are

salts of aryl hydroxycarboxylic acids as described above, preferably,salts of hydroxybenzoic acids having from 1 to 5 hydroxy groups, and most preferably salts of 2-hydroxybenzoic acid and of 2,5-dihydroxybenzoic acid. Preferred salts are salts of the acid moiety formed with $C_1$ - $C_5$ trialkyl amines, especially triethylamine, alkali metal hydroxides, especially sodium and potassium hydroxide, and quaternary ammonium hydroxides as described above.

By "hydroxycarboxylate compounds", "aryl hydroxycarboxylate compounds", and "-benzoate compounds" as used herein is meant the salt of the free acid, as well as the free acid-plus-base.

By "alkali metal hydroxides" is meant compounds of the formula MOH wherein M is an alkali metal such as lithium, sodium, potassium, cesium, or rubidium; potassium or sodium hydroxides are preferred herein. It is to be understood that the alkali metal hydroxides employed herein as bases are not particularly soluble in the organonitrile reaction medium. Apparently, enough alkali metal hydroxide does dissolve in the organonitrile to provide an effective amount of base for the esterification reaction. For this reason it is preferred to use alkali metal hydroxides which have been ground to an appropriate particle size to aid in dissolution in the organonitrile. The use of small particle sizes of alkali metal hydroxide is not critical to the invention, but does make the reaction more convenient.

By "organonitrile reaction medium" as used herein is meant a compound of the formula $R^8CN$ where $R^8$ is an organic substituent. Useful classes of organic nitriles

are aliphatic and aromatic nitriles wherein the cyano group is connected to an aliphatic group or an aromatic ring structure. An example of aromatic nitriles is benzonitrile, while examples of aliphatic nitriles include pentylnitrile and decylnitrile. Preferred aliphatic nitriles include lower alkylnitriles wherein the aliphatic substituent contains from 1 to 3 carbon atoms. The most preferred alkyl nitriles are acetonitrile and propionitrile.

By "esterification agent" as used herein is meant any compound of the formula $R^9X$ where $R^9$ is an organic substituent and X is any substituent described as a leaving group in the organic literature. Examples of esterification agents include organohalides, tosylates, and sulfates. Preferred esterification agents are the organohalides and the most preferred halides are the bromides and iodides. The preferred organohalides include compounds wherein the organic substituent is an aliphatic or cycloaliphatic alkyl group or an aralkyl group. The most preferred esterification agents are hexyl bromide, hexyl iodide, benzyl bromide, and benzyl iodide. Compounds such as sodium iodide, sodium bromide, and certain alkyl iodides which accelerate the rate of substitution reactions can be used to increase the rate of the esterification reaction, especially where somewhat less reactive organohalides such as aliphatic, cycloaliphatic, and aralkyl chlorides, such as hexyl or benzyl chloride, are used.

By "acylating agents" as used herein is meant compounds normally used in substitution reactions to interconvert various classes of compounds containing acyl groups. Preferred types of acylating agents are selected from acid anhydrides and acid halides. Preferred acid halides are the

acid chlorides and bromides. The most preferred acylating reactions are acetylating reactions wherein an acetyl group is substituted for the hydroxy group of the hydroxycarboxylate compound; accordingly, the most preferred of acylating agents used herein are acetic anhydride, acetyl chloride, and acetyl bromide.

By "contacting" as used herein is meant admixing the reactants in a reaction vessel.

"In the presence of" as used herein is meant that the organonitrile reaction medium and the other admixed components are in contact with a non-aqueous liquid base or a solid base. The base need only be soluble in the organonitrile reaction medium to a small extent so that it is only necessary that it be in intimate contact with the organonitrile reaction medium.

By "substantially water-free" herein is meant that water is not intentionally added to the organonitrile reaction medium. Some water may be present in the reaction medium, but usually only in trace amounts (less than about 2%). For example, the alkali metal hydroxides, especially LiOH, are somewhat hygroscopic and unavoidably carry some moisture into the system. Additionally, when the acylating agent is an acid anhydride, a higher water content can be tolerated than when the acylating agent is an acid halide.

By the term "comprising" as used herein is meant that various other compatible ingredients can be present in the reaction medium and other compatible reaction steps can be performed as long as the critical reactants or steps are present or performed. The term "comprising" thus encompasses and includes the more restrictive terms, "consisting essentially of" and "consisting of" which can be used not only to characterize the essential materials of hydroxycarboxylate compound, esterification agent, acylating agent, and organonitrile, but also to characterize the essential steps of esterification in an organonitrile reaction medium followed by acylation in the same reaction medium.

All percentages herein are by weight unless otherwise specified.

In general terms, Step 1 of the process herein is carried out by simply admixing either the esterification agent, the hydroxycarboxylic acid, the base, and the organonitrile, or, the esterification agent, the hydroxycarboxylic acid salt, and the organonitrile and allowing the reaction to proceed. Step 2 of the process is carried out by simply admixing the final reaction mixture produced by Step (1) with an acylating agent, and if the acylating agent is an acid anhydride, with an appropriate base, and allowing the reaction to proceed. If the acylating agent is an acid halide, base is optionally, but desirably, added in Step 2.

In the practice of this invention it is convenient to employ a stoichiometric amount of the hydroxycarboxylate compound with the esterification agent and acylating agent. Where the acylating agent is an acid anhydride it is convenient, in Step 2, to add a stoichiometric amount or a slight excess of base to the reaction mixture before admixing the acid anhydride; the purpose of the base in this step is to neutralize free acid produced by the acylation reaction of the anhydride and the hydroxycarboxylic acid ester. The organonitrile is generally used in a solvent amount that is

sufficient to dissolve the acid or acid salt. In some instances, the reactants are not entirely soluble in the organonitrile and a heterogeneous reaction mixture is formed. It does not appear to be necessary for the reaction mixture to be homogeneous and excellent yields of the esterified acylated hydroxycarboxylic acid are secured even under such conditions.

The nature of the hydroxycarboxylate compounds employed herein is not critical to the practice of the invention as long as there is a carboxyl group which can be esterified and a hydroxyl group which can be acylated. It will be appreciated, of course, that some hydroxycarboxylates will react more rapidly than others.

The esterification reaction mixture used in Step 1 is typically prepared by first admixing the organonitrile reaction medium and the hydroxycarboxylate compound. Where the free hydroxycarboxylic acid is being used, the admixing of base, acid, and organonitrile can be performed in any convenient order. The esterification agent is then added to begin the esterification reaction. The reaction mixture can be heated in any convenient manner. For example, all reactants can be admixed before heating the reaction mixture, or heating can take place during or before the admixture of reactants. The addition, admixture and heating of reactants takes place in accord with normal organic synthetic procedures according to the characteristics and quantities of the reactants.

The acylation reaction mixture of Step 2 is typically prepared by admixing the organonitrile reaction mixture produced by Step 1 and the acylating agent. The reaction mixture can be heated in any convenient manner;

for example, heating can take place before, during, or after admixing the reactants. The addition, admixture and heating of reactants takes place in accord with normal organic synthetic procedures according to the characteristics and quantities of the reactants.

When the acylation reaction of Step 2 is performed using an acid anhydride as the acylating agent, base is added to the final reaction mixture of Step 1. After adding the base, Step 2 is performed by adding the acid anhydride acylating agent and heating the reaction mixture according to standard acylation procedures.

The temperature at which Step 1 and Step 2 take place will vary depending upon the organonitrile and the reactants and can vary over a wide range. After admixture of the reactants for either Step 1 or 2, the temperature of the reaction mixture is maintained, conveniently, at the reflux temperature of the solvent. Reflux temperatures typically range from approximately 80° for acetonitrile to approximately 190°C for benzonitrile. The esterification and acylation reactions can be performed at temperatures less than the reflux temperature of acetonitrile, however, the length of time necessary to obtain completeness increases and temperature control is not as convenient.

It is to be understood that the velocity and completeness of the reaction Step 1 and 2 depend on the reactivity of the particular reactants chosen, but typically the overall esterification/alkylation reaction is complete after approximately 7 to 9 hours and no greater efficiency occurs by using reaction times longer than 30 hours.

Of course, the reaction steps can be performed under pressure to retain the benefits of the lower alkyl nitriles as solvents and to obtain the increased rate of reaction of higher temperatures.

The esterified acylated hydroxycarboxylate reaction products can be separated and purified by any standard methods. The products can be separated from the nitrile solvent, for example, by evaporating the solvent on a rotary evaporator or by reduced pressure distillation or by standard methods such as filtration, centrifugation, and the like. Purification of the product can involve standard steps and techniques such as washing with solvents, extraction with acidic, neutral, or basic solutions, and drying with various drying agents. Further purification steps can involve recrystallization from various solvents, filtration, and drying in vacuum desiccators or under reduced pressure.

The following examples illustrate the practice of the present invention in preparing acylated esters of hydroxycarboxylate compounds. The examples relate to the preparation of esterified/acylated hydroxybenzoic acid compounds, but the process herein can also be used to prepare esterified/acylated hydroxycarboxylic acid compounds from organic acids such as:

hydroxyacetic acid;

α-hydroxyisobutyric acid;

2-hydroxy-3-phenylpropionic acid;

9,10,16-trihydroxypalmitic acid;

α-hydroxyphenylacetic acid;

d,1-2-hydroxy-2-methyl succinic acid;

α-hydroxydiphenyl acetic acid;

2-hydroxycinnamic acid;

3-hydroxy-2-naphthoic acid;

3-hydroxypentanoic acid;

9,10-dihydroxyoctadecannoic acid; and

2,3-dihydroxycyclohexane carboxylic acid.

Esterification agents include compounds of from $C_1$ to $C_{20}$. Non-limiting examples of esterification agents include:

methyl iodide

benzyl chloride;

cyclopentyl bromide;

n-hexyl iodide;

cetyl sulfate;

α-phenylethyl bromide;

cyclohexyl tosylate.

Acylating agents include acid halides of from $C_1$ to $C_{20}$ and acid anhydrides of from $C_2$ to $C_{40}$. Non-limiting examples of acylating agents include:

2-naphthoyl bromide;

decanoyl chloride;

stearoyl bromide;

cyclohexanoyl chloride;

phthalic anhydride;

acetic anhydride;

isobutyric anhydride; and

α-napthoic anhydride.

## EXAMPLE I

### Esterification and Acylation of 2,5-dihydroxybenzoic acid

A five liter three-neck flask fitted with a water-cooled Friedrich's condenser, a 500 ml addition funnel, a thermometer, a teflon covered magnetic stirring bar, and a heating mantle is charged with 400 grams of 2,5-dihydroxybenzoic acid and 1200 ml of acetonitrile. The solution is heated to 40°C, and 360 ml of triethylamine is added in a slow stream (ca. 10 min.). The addition of triethylamine increases the temperature of the mixture to ca. 65°C. The addition funnel is rinsed with 200 ml of acetonitrile which is then added to the reaction mixture. To this reaction mixture is added 316 ml of benzyl bromide in a slow stream (ca. 10 min.). The addition funnel is washed with 200 ml of acetonitrile which is then added to the reaction mixture. The reaction mixture is heated to ca. 80°C, and stirred at that temperature for 24 hours.

The heating element is turned off and 522 ml of pyridine is run into the flask (pot temperature ca. 70°C). To this mixture, 516 ml of acetic anhydride is added dropwise (ca. 15 min.). The mixture is again heated to reflux ca. 80°C, and maintained at that temperature for 20 hours.

The flask is cooled, the solution is divided into three approximately equal portions and the acetonitrile is removed on a rotary evaporator. Each portion is then transferred to a separatory funnel with three liters of ethyl ether where it is sequentially washed twice with

750 ml of 1N HCl, once with 1 liter of $H_2O$, once with 500 ml of saturated aqueous sodium bicarbonate, once with 1 liter of water, and once with 500 ml of brine. The ether solution is dried with anhydrous $MgSO_4$. The drying agent is filtered and the ether evaporated to recover the product. The crude product is recrystallized from ethanol, filtered, and dried in a vacuum desiccator. Benzyl 2,5-diacetoxy-benzoic acid has a melting point of 71°C - 73°C and is obtained in approximately 80% yield.

EXAMPLE II

Octylpropyloxybenzoate

A three-neck flask fitted with a water-cooled condenser, an addition funnel, a thermometer, a magnetic stirring bar, and a heating mantle is charged with the salt produced from the reaction of 70 grams of 2-hydroxybenzoic acid and 46 ml of aniline, and 300 ml of benzonitrile. To this mixture is added 89 ml of octyl bromide. The addition funnel is washed with 40 ml of benzonitrile which is added to the reaction mixture. The reaction mixture is raised to reflux temperature and stirred for 14 hours. The heating element is turned off and 297 ml of tributylamine is run into the flask. To this mixture is added dropwise 46 ml of propanoic anhydride (ca. 15 min.). The mixture is again heated to reflux temperature for 20 hours. The flask is cooled, the benzonitrile is removed, and the product is purified.

## EXAMPLE III

### Propyl 3,4-diacetoxybenzoate

A three-neck flask fitted with a water-cooled condenser, an addition funnel, a thermometer, a magnetic stirring bar and a heating mantle is charged with 400 grams of 3,4-dihydroxybenzoic acid, 56 grams of potassium hydroxide, and 500 ml of acetonitrile. The solution is heated to 65°C. To this mixture 93 ml of propyl bromide is added quickly. The addition funnel is washed with 80 ml of acetonitrile which is added to the reaction mixture. The temperature of the reaction mixture is raised to reflux temperature and stirred for 24 hours. The heating element is turned off and 201 ml of pyridine is run into the flask. To this mixture is added dropwise 199 ml of acetic anhydride (*ca.* 15 min.). The mixture is again heated to reflux temperature for 20 hours. The flask is cooled, the acetonitrile is removed, and the product is purified.

EXAMPLE IV

Benzyl 2,5-diacetoxybenzoate

A three-neck flask fitted with a water-cooled condenser, an addition funnel, a thermometer, a magnetic stirring bar, and a heating mantle is charged with 308 grams of 2,5-dihydroxybenzoic acid and 930 ml of butyronitrile. The solution is heated to 40°C, and 476 ml of tributylamine is added in a slow stream (ca. 10 min.). This process increases the temperature of the mixture. The addition funnel is rinsed with 160 ml of butyronitrile which subsequently is added to the reaction mixture. To this mixture is added 235 ml of benzyl chloride in a slow stream (ca. 10 min.). The addition funnel is washed with 160 ml of butyronitrile which is added to the reaction mixture. The temperature of the reaction mixture is raised to reflux temperature and stirred for 24 hours. The heating element is turned off. To this mixture is added dropwise 150 ml of acetyl chloride (ca. 15 min.). The mixture is again heated to reflux temperature for 20 hours. The flask is cooled, the butyronitrile is removed, and the product is purified.

WHAT IS CLAIMED IS :

1. The process for preparing acylated esters of hydroxycarboxylate compounds comprising the steps of:

(1) contacting said hydroxycarboxylate compounds with an esterification agent in a substantially water free organonitrile reaction medium, and, thereafter ;

(2) acylating the product of step (1) by adding an acylating agent to the reaction medium of step (1).

2. A process according to Claim 1 wherein the organonitrile is selected from aliphatic and aromatic nitriles.

3. A process according to Claim 2 wherein the organonitrile is selected from acetonitrile and propionitrile.

4. A process according to Claim 3 wherein the esterification agent is selected from benzyl bromide, benzyl iodide, hexyl bromide, and hexyl iodide.

5. A process according to Claim 3 wherein the acylating agent is selected from acetic anhydride, acetyl chloride, and acetyl bromide.

6. A process according to Claim 1 wherein the hydroxycarboxylate compounds are selected from 2,5-dihydroxybenzoate compounds and 2-hydroxybenzoate compounds.

7. A process for preparing benzyl 2,5-diacetoxybenzoate comprising the steps of:

(1) contacting 2,5-dihydroxybenzoic acid with benzyl bromide or benzyl iodide in the presence of a base selected from potassium hydroxide, sodium hydroxide, trimethylamine, triethylamine, tripropylamine, tetramethylammonium hydroxide, tetraethylammonium hydroxide, tetrapropylammonium hydroxide, and tetrabutylammonium hydroxide in a substantially water free acetonitrile or propionitrile reaction medium, and, thereafter;

(2) acylating the product of step (1) by adding an acylating agent selected from acetic anhydride, acetyl chloride and acetyl bromide to the reaction medium of step (1).

8. A process for preparing benzyl 2,5-diacetoxybenzoate comprising the steps of:

(1) contacting a salt of 2,5-dihydroxybenzoic acid and a base selected from potassium hydroxide, sodium hydroxide, trimethylamine, triethylamine, tripropylamine, tetramethylammonium hydroxide, tetraethylammonium hydroxide, tetrapropylammonium hydroxide and tetrabutylammonium hydroxide with benzyl bromide or benzyl iodide in a substantially water free acetonitrile or propionitrile reaction medium, and thereafter;

(2) acylating the product of step (1) by adding an acylating agent selected from acetic anhydride, acetyl chloride, and acetyl bromide to the reaction medium of step (1)

9. A process for preparing hexyl 2,5-diacetoxybenzoate comprising the steps of:

(1) contacting a 2,5-dihydroxybenzoate compound with n-hexyl bromide or n-hexyl iodide in a substantially water free acetonitrile or propionitrile reaction medium, and, thereafter;

(2) acylating the product of step (1) by adding acetic anhydride, acetyl chloride, or acetyl bromide to the reaction medium of step (1).

10. A process for preparing benzyl 2-acetoxybenzoate comprising the steps of:

(1) contacting a 2-hydroxybenzoate compound with benzyl bromide or benzyl iodide in a substantially water free acetonitrile or propionitrile reaction medium, and, thereafter;

(2) acylating the product of step (1) by adding acetic anhydride, acetyl chloride, or acetyl bromide to the reaction medium of step (1).

European Patent Office

**EUROPEAN SEARCH REPORT**

0002872

Application number

EP 78 20 0388

## DOCUMENTS CONSIDERED TO BE RELEVANT

| Category | Citation of document with indication, where appropriate, of relevant passages | Relevant to claim |
|---|---|---|
| EP | FR - A - 2 359 110 (ROHM AND HAAS)<br>* Page 3, line 11 - page 4, line 14; pages 5-6, example 1; page 2, lines 14-16 *<br>& DE - A - 2 732 144<br>-- | 1-3,5,6 |
| EP | FR - A - 2 374 292 (PROCTER & GAMBLE)<br>* Page 6, example 1 *<br>& DE - A - 2 755 198<br>& NL - A - 77 13896<br>& BE - A - 861 889<br>---- | 1-8 |

CLASSIFICATION OF THE APPLICATION (Int. Cl.²)

C 07 C 69/90
67/10
67/14
67/08
69/88

TECHNICAL FIELDS SEARCHED (Int.Cl.²)

C 07 C 69/90

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-03-1979 | KINZINGER |